(19) Europäisches Patentamt
European Patent Office
Office européen des brevets

(11) Publication number: 0 015 605 B1

(12) EUROPEAN PATENT SPECIFICATION

(45) Date of publication of patent specification: 09.02.83

(51) Int. Cl.$^3$: C 07 C 127/19, A 01 N 47/30

(21) Application number: 80200141.2

(22) Date of filing: 19.02.80

# (54) Novel urea derivative, a process for its preparation, herbicidal composition containing it, a method of combating undesired plant growth using it.

(30) Priority: 01.03.79 US 16671

(43) Date of publication of application: 17.09.80 Bulletin 80/19

(45) Publication of the grant of the patent: 09.02.83 Bulletin 83/6

(84) Designated Contracting States: BE DE FR GB IT NL

(56) References cited:
US - A - 2 655 447

(73) Proprietor: SHELL INTERNATIONALE RESEARCH MAATSCHAPPIJ B.V.
Carel van Bylandtlaan 30
NL-2596 HR Den Haag (NL)

(72) Inventor: Pilgram, Kurt Hans Gerhard
2607 Warwick Lane
Modesto, California 95350 (US)
Inventor: Bozarth, Gene Allen
1235 Magnolia
Modesto, California 95350 (US)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 015 605 B1

Novel urea derivative, a process for its preparation, herbicidal composition containing it, a method of combating undesired plant growth using it

The invention relates to a novel urea derivative, a process for its preparation, herbicidal composition containing it, a method of combating undesired plant growth using it.

US Patent Specification No. 2655447 discloses an extremely large class of urea derivatives which can be used as herbicides. Said specification, however, contains no suggestion that such ureas have any selective action between weeds and crops. Surprisingly, it has now been found that a specific urea derivative within the scope of said specification but not specifically disclosed therein, has useful selective herbicidal properties.

The invention provides the compound N-(2-methoxy-3-propylphenyl)-N'-methylurea. The invention also provides a process for the preparation of said compound, characterised in that a compound of the general formula $CH_3X$ is reacted with a compound of the general formula

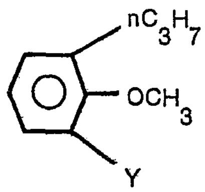

in which one of X and Y represents an isocyanate group and the other represents an amino group.

Suitably the reaction is carried out in the presence of an inert solvent, for example an ether such as diethyl ether or tetrahydrofuran. Preferably the reaction is carried out at a temperature in the range of from 0 to 100°C, especially 15 to 75°C. Room temperature is generally convenient. The reaction is suitably performed in the presence of a base. This may be an excess of one of the reactants, especially when using methylamine as a reactant, or an additional base, for example an amine, may be added.

It has been found that N-(2-methoxy-3-propylphenyl)-N'-methylurea possesses useful herbicidal properties, controlling both broad-leaved and grassy weeds. In particular, it has been found that when it is applied pre-emergence (applied to the soil before the plants sprout) it kills such weeds at dosages at which it is only slightly toxic to wheat. It thus is of interest for controlling weeds in plantings of wheat. The invention therefore also provides a herbicidal composition which comprises the compound according to the invention together with a suitable carrier, and also provides a method of combating undesired plant growth at a locus, which comprises applying to the locus the compound or a composition according to the invention. In particular, the compound or composition may be applied to a crop area sown with wheat, before the emergence of the wheat.

The amount of the compound according to the invention, to be used in combating undesirable vegetation will naturally depend on the condition of the vegetation, the degree of activity desired, the formulation used, the mode of application, the climate, the season of the year, and other variables. Recommendations as to precise amounts are, therefore, not possible. In general, however, application to the locus to be protected of from 0.1 to 10.0 kilograms per hectare of said compound will be satisfactory.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution.

A surface-active agent may be an emulsifying agent, a dispersing agent or a wetting agent; it may be nonionic or ionic. Examples of suitable surface-active agents include the sodium or calcium salts of

polyacrylic acid and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$—10% w of active ingredient. Granules are usually prepared to have a size between 10 and 100 BS mesh (1.676—0.152 mm), and may be manufactured by agglomeration or impregnation techniques. Generally, granules will contain $\frac{1}{2}$—25% w active ingredient and 0—10% w of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing insecticidal, herbicidal or fungicidal properties.

The following Examples illustrate the invention. The identities of intermediates and the final product in Examples 1 and 2 were confirmed by chemical and spectral analyses.

## Example 1

12.0 g of sodium hydride (50% in mineral oil) were added, in portions, to a stirred solution of 68.9 g of 2-propylphenol in 350 ml of dimethyl sulphoxide, the temperature of the mixture being held below 40°C. The mixture was then stirred for one hour at 40°C, when a solution of 78.1 g of methyl iodide in 50 ml of dimethyl sulphoxide was slowly added, the temperature of the mixture being held below 60°C. The resulting mixture was held overnight at room temperature, then poured into water and extracted with diethyl ether. The extract was washed with 10% sodium hydroxide solution, then with water, and dried, and the solvent was evaporated under reduced pressure to give 1-methoxy-2-propylbenzene (IA), as a liquid.

A mixture of 67.2 g of IA, 83.8 g of silver nitrate and 350 ml of acetonitrile was stirred at room temperature until the solids were in solution. The solution was cooled to 5°C and slowly dripped into a solution of 38.7 g of acetyl chloride in 50 ml of acetonitrile. The resulting mixture was stirred for two hours at 0—5°C, and was then allowed to warm slowly to room temperature and stirred overnight. The mxiture was then cooled to 5°C and 750 ml of water was added. The resulting mixture was allowed to warm to room temperature, 500 ml of water were added and the resulting mixture was filtered. The filtrate was extracted with diethyl ether. The extract was washed with 10% sodium hydroxide solution, then with water, dried, and filtered, and the solvent was evaporated under reduced pressure to give a product (IB) containing two significant components.

20.0 g of IB dissolved in tetrahydrofuran were treated with hydrogen (60 psig, approximately 4 atmospheres gauge) in a Parr apparatus, using Raney nickel as catalyst. The resulting mixture was filtered to give a solution (IC) containing two significant components.

3.2 g of methyl isocyanate and 8—10 drops of triethylamine were added to one-half of IC at room temperature and the mixture was held overnight. The solvent was then evaporated under reduced pressure. Diethyl ether was added to the residue and the resulting mixture was filtered. The solvent was evaporated from the filtrate under reduced pressure and the residue was chromatographed over silica gel, using a 4/30/66 v/v/v mixture of tetrahydrofuran, ethyl acetate and hexane as eluent. Two products

were isolated and identified by appropriate chemical and spectral analyses: N-(2-methoxy-3-propylphenyl)-N'-methylurea as a solid, mp 79—81 °C, and an isomer.

Example 2

200 g of allyl chloride were added over a two-hour period to a mixture of 600 ml of water, 80 g of sodium hydroxide and 278 g of 2-nitrophenol. The mixture was refluxed for two hours, and was then extracted with a 3:1 v/v mixture of hexane and toluene. Evaporation of the solvent gave allyl 2-nitrophenyl ether, as a light yellow liquid, bp: 96—98°C (0.15 Torr.).

54 g of that product was heated at 180°C for two hours, then the temperature was gradually raised. At 200°C an exothermic reaction occurred, and the temperature of the mixture rose to 250°C and was held there by cooling. The resulting mixture was purified by silica gel chromatography to give 2-allyl-6-nitrophenol, as a yellow oil.

30 g of the phenol were mixed with 75 ml of methanol to which had been added 10 g of sodium hydroxide dissolved in 10 ml of water. Two equivalents of sodium hydroxide were added, and then two equivalents of dimethyl sulphate were added drop by drop to the stirred mixture. The mixture was then refluxed for two hours. Workup of the resulting mixture gave methyl (2-allyl-6-nitro)phenyl ether, as a dark yellow oil.

28.5 g of the ether was reduced in a Parr shaker, three hours at 60 psig hydrogen pressure, tetrahydrofuran solvent, palladium-on-charcoal catalyst, to give 2-methoxy-3-propylaniline, as a liquid.

Excess phosgene was added to a solution of the aniline in 400 ml of ethyl acetate and the mixture was refluxed to give 2-methoxy-3-propylphenylisocyanate.

Excess methylamine was added to a solution of 10 g of the isocyanate in 75 ml of diethyl ether. An exothermic reaction occurred and a solid product precipitated. The mixture was filtered and the solid product was purified to give the desired product as a white solid, mp: 74—77°C.

Example 3 to 5

In the following examples, the species of plants tested were:

Barnyard grass — *Echinochloa crus-galli*
Crabgrass — *Digitaria sanguinalis*
Downy brome — *Bromus tectorum*
Giant foxtail — *Setaria faberii*
Wild oats — *Avena fatua*
Yellow foxtail — *Setaria lutescens*
Hemp sesbania (coffeeweed) — *Sesbania exaltata*
Jimsonweed — *Datura stramonium*
Ivyleaf morningglory — *Ipomoea hederacea*
Mustard — *Brassica kaber*
Redroot pigweed — *Amaranthus retroflexus*
Prickly sida — *Sida spinosa*
Sicklepod — *Cassia obtrusifolia*
Velvetleaf — *Abutilon theophrasti*
Garden cress — *Lepidium sativum*
Corn — *Zea maize* (DeKalb X363)
Cotton — *Gossypium hirsutem* (Acala SJ—2)
Rice — *Oryza sativa* (Calrose)
Grain sorghum — *Sorghum vulgare* (Pioneer 265)
Soybean — *Glycine max* (amxoy 71)
Wheat — *Triticum aestivum* (Cajeme 71)

Example 3

The pre-emergence herbicidal activity of the compound of the invention was evaluated by planting seeds of barnyard grass, garden cress, downy brome, velvetleaf, yellow foxtail, and sicklepod in test tubes, nominally measuring 25 x 200 millimeters, containing soil treated with the test compound at the rates of 0.1 and 1 milligram per tube, given in Table I as Rates I and II, respectively. The planted soil was held under controlled conditions of temperature, moisture, and light for 9 to 10 days. The amount of germination and growth in each tube were evaluated on a 0 to 9 scale, 0 rating indicating no effect, 9 death of the seedlings or no germination.

The post emergence activity of the test Compound was evaluated by spraying 10-day old crabgrass plants, 13-day old pigweed plants, 6-day old downy brome plants, 9-day old sicklepod plants to runoff with a liquid formulation of the test compound at the rates of 2.4 milliliters of an 0.025% solution (designated Rate I in Table I) and 2.4 milliliters of an 0.25% solution (designated Rate II in Table I). The sprayed plants were held under controlled conditions for 7 to 8 days and the effect of the test compound was then evaluated visually, the results being rated on the 0 to 9 scale described above.

The results of the tests are summarized in Table I.

TABLE I

HERBICIDE SCREEN RESULTS

| Pre-emergence (Soil) | | | | | | Post-emergence (Foliar) | | | | | |
|---|---|---|---|---|---|---|---|---|---|---|---|
| Barnyard Grass | Garden Cress | Downy Brome | Velvetleaf | Yellow Foxtail | Sicklepod | Crabgrass | Pigweed | Downy Brome | Velvetleaf | Yellow Foxtail | Sicklepod |
| ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ | ${}^{I}{}_{II}$ |
| ${}^{4}{}_{6}$ | ${}^{6}{}_{9}$ | ${}^{4}{}_{7}$ | ${}^{6}{}_{9}$ | ${}^{6}{}_{7}$ | ${}^{7}{}_{8}$ | ${}^{2}{}_{9}$ | ${}^{3}{}_{9}$ | ${}^{2}{}_{3}$ | ${}^{4}{}_{9}$ | ${}^{5}{}_{7}$ | ${}^{2}{}_{7}$ |

Example 4

The pre-emergence herbicidal activity of the compound of the invention was further determined with respect to several common species of weeds, by spraying a formulation of the test compound on the soil in which seeds of the weeds had been sown. In each series of tests, the plants were grown in narrow trays and sprayed with the test compound. The solution of the test compound was sprayed over the tray, from one end to the other, the concentration of the test compound in the formulation varying logarithmically from a higher value at one end of the band to a lower value at the other end of the band. The effect of the test compound was evaluated visually and reported as the nominal rate of application, in pounds of test compound per acre of the soil band, at which 90% inhibition of the growth of the weeds occurred, this being referred to as the 90% growth inhibition or $GID_{90}$ dosage. Results of the test, as well as the weed species involved, are set out in Table II.

TABLE II

| | Barnyard Grass | Downy Brome | Yellow Foxtail | Crab-grass | Velvet-leaf | Wild Mustard | Pig-weed | Prickly Sida |
|---|---|---|---|---|---|---|---|---|
| $GID_{90}$ | 1.0 | 1.3 | 1.9 | 2.5 | 1.0 | 0.8 | 1.5 | <0.55[a)] |

a) The symbol < means "less than".

Example 5

The pre-emergence activity of the compound of the invention was further determined with respect to a number of crop plants and common species of weeds, using the procedure described in Example 4 except that the test compound was applied at three different fixed dosages of 0.5, 1.0 and 2.0 pounds of test compound per acre, and the results were evaluated with reference to a scale:

0 = no visible injury
1—3 = slight (increasing) injury
4—6 = moderate (increasing) injury
7—8 = severe injury
9 = complete kill of the plant.

The results of the tests are reported in Table III.

TABLE III

| Plant Species | Rating of effect at indicated dosage | | |
|---|---|---|---|
| | 0.5 | 1.0 | 2.0 |
| Corn | 0 | 0 | 0 |
| Cotton | 0 | 3 | 3 |
| Rice | 0 | 2 | 7 |
| Grain Sorghum | 2 | 3 | 4 |
| Soybean | 2 | 7 | 7 |
| Wheat | 0 | 0 | 0 |
| Barnyard Grass | 5 | 7 | 8 |
| Crabgrass | 0 | 4 | 6 |
| Downy Brome | 0 | 5 | 7 |
| Giant Foxtail | 0 | 5 | 7 |
| Wild Oats | 6 | 8 | 9 |
| Yellow Foxtail | 0 | 3 | 7 |
| Hemp sesbania (Coffeeweed) | 0 | 0 | 0 |
| Jimsonweed | 8 | 0 | 0 |
| Morningglory | 0 | 6 | 6 |
| Mustard | 7 | 8 | 9 |
| Pigweed | 3 | 9 | 9 |
| Prickly sida | 3 | 9 | 9 |
| Sicklepod | 0 | 7 | 7 |
| Velvetleaf | 0 | 5 | 9 |

## Claims

1. N-(2-Methoxy-3-propylphenyl)-N'-methylurea.

2. A process for the preparation of the compound claimed in claim 1, characterized in that a compound of the general formula $CH_3X$ is reacted with a compound of the general formula

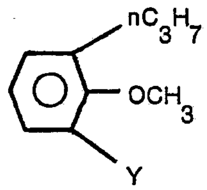

in which one of X and Y represents an isocyanate group and the other represents an amino group.

3. A process as claimed in claim 2, characterized in that it is carried out at a temperature in the range of from 0 to 100°C.

4. A process as claimed in either claim 2 or claim 3, characterized in that X represents an amino group and the methylamine is present in excess over the isocyanate.

5. A process as claimed in either claim 2 or claim 3, characterized in that Y represents an amino group and the reaction is carried out in the presence of an additional base.

6. A herbicidal composition which comprises the compound claimed in claim 1 together with a suitable carrier.

7. A composition as claimed in claim 6 which comprises at least two carriers, at least one of which is a surface-active agent.

8. A method of combating undesired plant growth at a locus, which comprises applying to the locus the compound claimed in claim 1 or a composition as claimed in either claim 6 or claim 7.

9. A method as claimed in claim 8 in which the compound or composition is applied to a crop area sown with wheat, before the emergence of the wheat.

**Revendications**

1. N-(2-méthoxy-3-propylphényl)-N'-méthylurée.

2. Un procédé pour la préparation du composé revendiqué dans la revendication 1, caractérisé en ce qu'on fait réagir un composé de la formule générale $CH_3X$ avec un composé de la formule générale

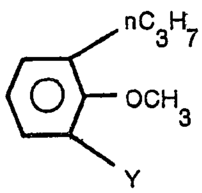

dans lesquelles un des substituants X et Y représente un groupe isocyanate et l'autre représente un groupe amino.

3. Un procédé selon la revendication 2 caractérisé en ce qu'il est mis en oeuvre à une température comprise entre 0 et 100°C.

4. Un procédé selon l'une des revendications 2 et 3, caractérisé en ce que X représente un groupe amino et la méthylamine est présente en excès par rapport à l'isocyanate.

5. Un procédé selon l'une des revendications 2 et 3, caractérisé en ce que Y représente un groupe amino et la réaction est conduite en présence d'une base supplémentaire.

6. Une composition herbicide qui comprend le composé selon la revendication 1 en même temps qu'un véhicule approprié.

7. Une composition selon la revendication 6 qui comprend au moins deux véhicules, dont l'un au moins est un agent tensio-actif.

8. Une méthode de lutte contre la croissance de plantes indésirables en un lieu, qui comprend l'application en ce lieu du composé selon la revendication 1 ou d'une composition selon l'une des revendications 6 et 7.

9. Une méthode selon la revendication 8, dans laquelle on applique le composé ou la composition à une zone cultivée où on a semé du blé, avant l'émergence du blé.

**Patentansprüche**

1. N-(2-Methoxy-3-propylphenyl)-N'-methylharnstoff.

2. Verfahren zur Herstellung der Verbindung nach Anspruch 1, dadurch gekennzeichnet, daß eine Verbindung der allgemeinen Formel $CH_3X$ umgesetzt wird mit einer Verbindung der allgemeinen Formel

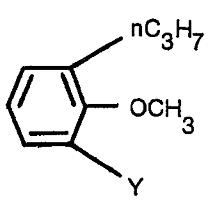

wobei einer der Reste X und Y eine Isocyanatgruppe und der andere eine Aminogruppe bedeutet.

3. Verfahren nach Anspruch 2, dadurch gekennzeichnet, daß es bei einer Temperatur in Bereich von 0 bis 100°C durchgeführt wird.

4. Verfahren nach Anspruch 2 oder Anspruch 3, dadurch gekennzeichnet, daß X eine Aminogruppe bedeutet und das Methylamin in einem Überschuß gegenüber dem Isocyanat vorhanden ist.

5. Verfahren nach Anspruch 2 oder 3, dadurch gekennzeichnet, daß Y eine Aminogruppe bedeutet und die Reaktion in Gegenwart einer Zusätzlichen Base durchgeführt wird.

6. Herbicides Mittel, umfassend die Verbindung nach Anspruch 1 zusammen mit einem geeigneten Träger.

7. Mittel nach Anspruch 6, umfassend mindestens 2 Träger, von denen zumindest einer ein oberflächenaktives Mittel ist.

8. Verfahren zur Bekämpfung von unerwünschtem Pflanzenwachstum an einer Stelle, umfassend das Aufbringen der Verbindung nach Anspruch 1 oder eines Mittels nach Anspruch 6 oder 7 auf die Stelle.

9. Verfahren nach Anspruch 8, wobei die Verbindung oder das Mittel auf ein Feld aufgebracht wird, in dem Weizen gesät ist, vor dem Auflaufen des Weizens.